(19) Europäisches Patentamt / European Patent Office / Office européen des brevets

(11) **EP 3 203 966 B1**

(12) **EUROPEAN PATENT SPECIFICATION**

(45) Date of publication and mention of the grant of the patent:
**21.06.2023 Bulletin 2023/25**

(21) Application number: **15784924.1**

(22) Date of filing: **01.10.2015**

(51) International Patent Classification (IPC):
*A61B 5/03* (2006.01)     *A61B 5/026* (2006.01)
*A61B 5/11* (2006.01)     *A61B 5/0215* (2006.01)
*A61B 5/341* (2021.01)     *A61B 5/352* (2021.01)

(52) Cooperative Patent Classification (CPC):
**A61B 5/031; A61B 5/0215; A61B 5/026;**
**A61B 5/11; A61B 5/1102; A61B 5/341;**
**A61B 5/352; A61B 5/6892;** A61B 5/002;
A61B 5/746

(86) International application number:
**PCT/CZ2015/000114**

(87) International publication number:
**WO 2016/055036 (14.04.2016 Gazette 2016/15)**

(54) **DEVICE AND METHOD FOR MEASUREMENT OF INTRACRANIAL PRESSURE**

VORRICHTUNG UND VERFAHREN ZUR MESSUNG DES INTRAKRANIALEN DRUCKS

DISPOSITIF ET PROCÉDÉ DE MESURE DE LA PRESSION INTRACRÂNIENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.10.2014 CZ 20140696**

(43) Date of publication of application:
**16.08.2017 Bulletin 2017/33**

(73) Proprietor: **LINET spol. s r.o.**
**274 01 Slany (CZ)**

(72) Inventors:
• **SEBA, Petr**
**517 01 (CZ)**
• **STUDNICKA, Filip**
**500 03 Hradec Králové (CZ)**

(56) References cited:
DE-A1- 3 130 326     US-A1- 2006 079 773
US-A1- 2013 289 422

• **Hannu Sorvoja: "NONINVASIVE BLOOD PRESSURE PULSE DETECTION AND BLOOD PRESSURE DETERMINATION", ACTA UNIVERSITATIS OULUENSIS, 8 December 2006 (2006-12-08), XP55240250, Retrieved from the Internet: URL:http://herkules.oulu.fi/isbn9514282728 /isbn9514282728.pdf [retrieved on 2016-01-11]**
• **LINET Group SE: "LINET introduces groundbreaking method for monitoring intracranial pressure", , 13 October 2014 (2014-10-13), pages 1-3, XP55239403, Retrieved from the Internet: URL:http://www.linet.com/-/media/Files/Web site/linet/news/20141013_Press_Release_Gro undbreaking_method.ashx [retrieved on 2016-01-07]**

**EP 3 203 966 B1**

**Description**

Technical field of the invention

The invention concerns a device and methods for non-invasive measurement of intracranial pressure (ICP). The monitoring device consists of a mat with one or more piezoelectric elements, and of a device for R-wave detection in the ECG signal and optionally an invasive measuring device for determining arterial blood pressure (ABP),

Background of the invention

Measurement of intracranial pressure (ICP) is very important for many clinical and diagnostic methods. ICP monitoring is crucial namely for patients at the neurological department and for polytrauma patients, e.g., after an accident. Increased ICP may indicated serious, life-threatening brain injury and practically always requires immediate surgery. High ICP may indicate for example a tumor, edema, acute liver failure and other life-threatening conditions. At the same time, low ICP is also physiologically dangerous, and is accompanied by nausea, migraines or visual impairment.
The most commonly used and the only accurate method of ICP measurement is currently an invasive method, during which pressure sensors are introduced to the brain tissue and the physician needs to drill holes into the patient's skull. This methods poses risks for the patient in terms of health and following recovery, as well as the risk of infection.
Non-invasive methods known so far include measurements of intraocular pressure, otoacoustic emissions or tympanometry, visually stimulated evoked potentials or transcranial Doppler measurement of blood flow. Unfortunately, these methods do not guarantee sufficient accuracy and reliability of measurements.
One non-invasive method used for ICP measurement is described in application US2013289422, including the description of the ICP measuring device based on the relation between the pressure inside a carotid artery and the flow/flow speed of blood inside a carotid artery. The blood flow is measured using a piezoelectric sensor, which is attached to the carotid artery. Deduction of the ICP value is based on the shape of the pulse wave measured using a piezoelectric sensor.
Another approach to ICP measurement is described in document US6761695, which describes a device for determining ICP consisting of a pressure sensor attached to the head of the measured subject. The output of the sensor includes an ICP component and blood pressure component. Then the processor subtracts the blood pressure value from the output signal in the same phase, which yields ICP.
Another possible approach and device for ICP monitoring are described in US2009012430. This device is primarily intended for monitoring of the bloodstream in the brain and it can be used to also determine ICP. The principle consists in an injection of micro-bubbles into the patient's blood stream and following assessment of turbulent flow caused by these micro-bubbles. These vibrations are recorded by a field of accelerometers attached to the patient's head.
Another method for determining ICP is described in US2010049082, which describes a procedure where using a number of vital parameters ($pCO_2$, $pO_2$, blood pressure, blood flow speed...) it is possible to statistically assess the assumed ICP. This method includes two phases of the process: learning, during which data are collected, and simulation, during which data are assigned to potential ICP models. The disadvantage of this procedure is the relatively irrelevant output values that may not always correspond to the truth.
Unlike the above described procedures, the procedure described in US8366627 is significantly more complex and reliable. It uses a pressure sensor (tonometer or catheter) for blood pressure monitoring to calculate the ICP value; the calculation model contains parameters of resistance, submission, blood pressure and blood flow. There are also solutions consisting of a sensor attached to the patient's hand using a band, such as described in US2013085400, which describes a sensor, whose output signal is processed and transferred using mathematical operations to a frequency spectrum and its components. These operations include namely the Fourier transform, Fast Fourier transform or Wavelet transform.
Another alternative approach to measurement of a brain parameter, not directly ICP but a similar parameter, i.e., the blood pressure in the temporal artery, is discussed in US2011213254, which describes a measuring device similar to headphones, which is in contact with the ear but the sensor is placed on across a temporal artery, where it measures pulsation and deduces the blood pressure in the temporal artery.
A further intracranial pressure measurement configuration according to the state of the art is for instance described in DE3130326A1.

Summary of the invention

The specified problems and shortcomings of the stated methods and approaches to calculation of intracranial (ICP) pressure are solved by a device for ICP monitoring according to claim 1 comprising a measuring mat containing optionally at least one piezoelectric sensor. This mat is placed under the head of the patient. Other optional components of this device include a device for heart rate measurement and a sensor for invasive measurement or arterial blood pressure (ABP).

The measuring mat detects micro movements and mechanical vibrations of the head (i.e. in another words mechanical manifestations of the head) that are caused by the hemodynamics of the patient's blood circulation, thanks to which the pulse wave is reflected in the bloodstream inside the head. Furthermore, ECG is used to detect the R-wave. Another part of the device is a sensor for invasive measurement of ABP. ICP is then calculated from a relation using the time delay of the reflected pulse wave in relation to the moment of the detected R-wave.

An experimental study discovered and verified that relative changes of IPC may be measured even without the necessity to use invasive ABP measurement or to detect the R-wave. The method is based on the detection of a sequence of pulse waves and their reflection in relation to individual pulses and their mutual time delay.

Description of the drawings

Fig. 1a, 1b and 1c schematically represent the device and its components. Fig. 2 shows the sensor mat for ICP measurement. Fig. 3 schematically depicts the vascular system.

Fig. 4 represents measurement using the invention in comparison with invasive ICP measurement. Fig. 5 shows the data matrix of ECG signal with synchronized R-waves.

Fig. 6 shows the data matrix of the signal from the measuring mat. Fig. 7 shows the time match of the ICP peak with one of the peaks of head movement. Fig. 8 represents the data matrix of ECG signal with highlighted mechanical manifestations.

Description of the preferred embodiments

The device for non-invasive monitoring of intracranial pressure 1 (ICP) includes a measuring mat 2, processor unit 3, device for recording electrical activity of the heart 4 (ECG), device for invasive measurement of arterial blood pressure 5 (ABP), imaging device 6 and network connector 7.

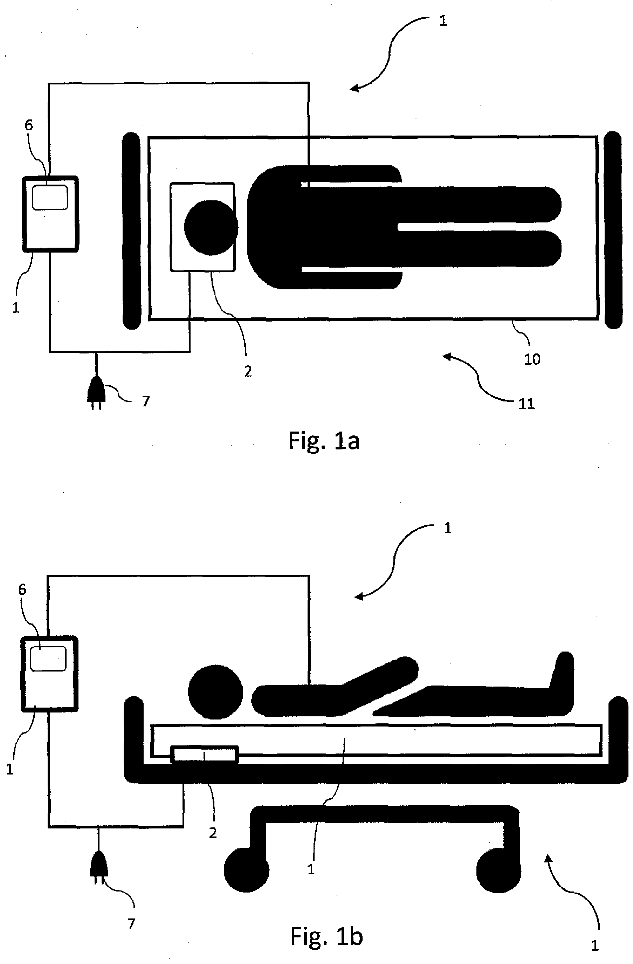

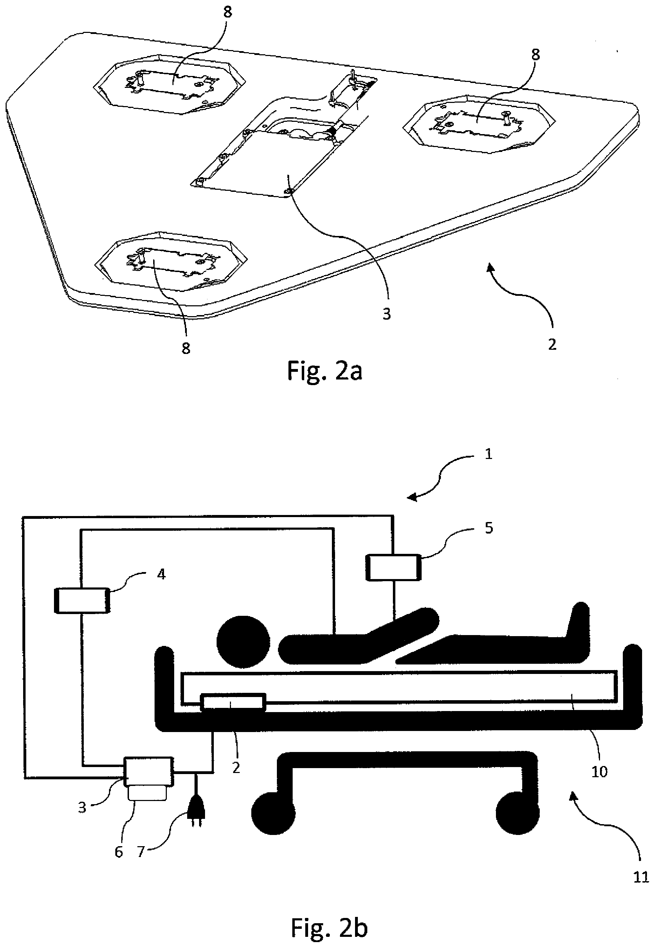

The measuring mat 2 includes at least one piezoelectric sensor 8 and is located in a suitable design under the patient's head in the head support, as shown in Fig. 1a. It is suitable to use the piezoelectric sensor with a third conductive capacity electrode as described in patents EP3054837 or US10376216 or CN105813551 which are the National Phases of the International Application PCT/CZ2014/000112 filed on October 8, 2014, which claims priority to Czech Application PV2013-781 filed on October 8, 2013, currently also being a patent CZ308222 as it provides additional information about the patient. The head support may also include an extension 9 which is adjusted to keep the patient's head in one position and to prevent self-positioning of the patient's head to the sides. It is suitable to cover the measuring mat 2 with a material that ensures better comfort during handling and which has better hygienic properties. Alternatively the measuring mat 2 may be placed under the mattress 10 on the hospital bed 11 under the patient's head, as depicted in Fig. 1b. In advantageous arrangement three sensors 8 are connected in the measuring mat 2, as indicated in Fig. 2; however, for purposes of monitoring of long-term health trends, one sensor 8 in the measuring mat 2 is fully sufficient, as the signals coming from three sensors 8 are similar. Mechanical manifestations of the bloodstream dynamics in alternative embodiments may be detected using for instance a piezoresistive sensor or accelerometer; alternatively the bloodstream dynamics may be monitored optically or using a different suitable method.

The measuring device 1 also includes a device for monitoring of electrical activity of the heart 4 (ECG), sensor for invasive measurement of arterial blood pressure 5 (ABP). It is suitable to extend these functions by a standard patient monitor 12 with data output. The output signal is preferably digital; however, analog signal can also be used. If analog output signals are used, it is necessary to use A/D converters 13. Experts familiar with processing of analog signals can design several possible connections of the A/D converter 13 in order for the processor unit 3 to be able to correctly assess signals and calculate ICP. At the same time, any expert familiar with biosignal can use other suitable equipment for monitoring of electric activities of the heart, such as vectorcardiograph (VCG). Alternatively, ballistocardiographic signal can also be used. In an alternative embodiment it is possible to use non-invasive ABP measurement; however, preferred embodiment assumes non-stop monitoring of the patient in an intensive care unit or in an anesthetics and resuscitation department, where ABP is normally measured invasively; this is why this invention is described on the example of an invasive sensor 5 for ABP measurement.

The processor unit 3 is preferably located inside the measuring mat 2, as indicated in Fig. 2a. In an alternative embodiment (Fig. 2b) it can be located outside the measuring mat 2, as a separate module with its independent display 6; ECG 4 and invasive ABP measuring device 5 may also be separate. All recorded signals are then transferred to the processor unit 3. In an alternative embodiment, the processor unit 3 may be a part of a specialized patient monitor 12. In this case the signal from the measuring mat 2 is conducted directly to the specialized patient monitor 12, where the processor unit 3 assesses ICP and displays it on the screen 6 of monitor 12.

Processor unit 3 is connected with sensors 8, ECG 4 and ABP sensor 5. Signals from these sensors 8 are processed by the processor unit 3 and the processed data are then sent to the displaying device 6. This displaying device 6 may be placed separately, as shown in Fig. 2b, or preferably it can be a part of the patient monitor 12. In this case the data are connected to the connector for external output signal. In this embodiment there is no need to have a device for monitoring of electrical activity of the heart 4 (ECG) and a sensor for invasive measurement of arterial blood pressure 5 (ABP) as the signals generated by these devices are taken directly from the measuring mat 2 and measurement is thus non-invasive.

The displaying device 6 may display current values, trends, mean values, ICP, ECG, ABP, alternatively other vital functions of the patient in case of the patient monitor 12. The displaying device 6 can be advantageously connected with the hospital system for collection of patient data 15. The displaying device 6 can also display critical states, when the value of the displayed parameter is outside the predefined limit values. Limit values can be adjusted manually in accordance with individual needs of the patient. Critical situations may also be detected if the current ICP value deviates from the long term average. Notification of these situations may again be sent to the system for collection of patient data 15, which then further distributes the information, alternatively information on selected critical situations may be sent directly to the medical staff.

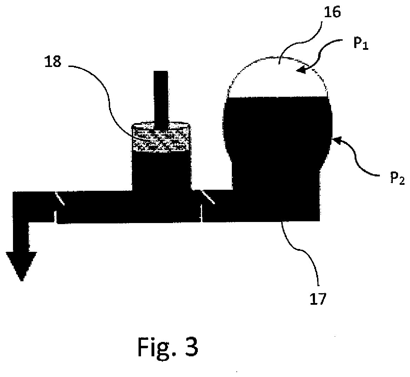

The ICP calculation method is based on the existing relation between pressure inside the cranial cavity 16 and the bloodstream hemodynamics 17. This mutual relationship is manifested for example by synchronous changes of ICP and heart activity (as described for instance in article by Wagshul M. et al. (2011) The pulsating brain: A review of experimental and clinical studies of intracranial pulsatility, Fluids and Barriers of the CNS, 8:5). From the mechanical point of view, synchronized ICP and ABP oscillation occurs and it is caused by changes of ABP as well as the volume of blood in brain arteries and vessels. The brain volume changes proportionally to ABP. If the brain were not located in the cranial cavity 16, clear pulsations would be visible. In fact the brain is stored in cerebrospinal fluid, an incompressible fluid, and is enclosed in a hard shell (skull). Increased ABP leads to brain swelling and to higher ICP. When ABP and ICP are locally balanced, the brain cannot increase its volume any more, as it cannot receive any more blood due to the fact that the pressure of blood entering the brain is no longer higher than ICP and the entering pulse wave is reflected. This situation is schematically represented in Fig. 3, where ICP corresponds to $p_1$ and ABP corresponds to $p_2$. The heart 18 is represented as a pumping piston that pumps blood. This situation is commonly described using the simple Windkessel hydrodynamic model. In common practice the Windkessel model is used to determine the aorta elasticity. This model is solved using differential equations, where the heart 18 is represented as the pulse source and input parameters are flexibility of arteries and vascular resistance. This model enables calculation and description of the pulse wave. Windkessel is described in detail for instance in Westerhof N. et al. (2008) The arterial Windkessel, DOI 10.1007/s1 1517-008-0359-2.

Analytical solution of these differential equations is impossible, which is why they need to be solved numerically. This model is used for pulse wave reflection in the head. On the basis of physical considerations it may be deduced that the elasticity of bloodstream in the brain C is inversely proportional to ICP and the following equation I therefore applies

$$ICP \sim \frac{1}{C} \qquad \text{(I)}$$

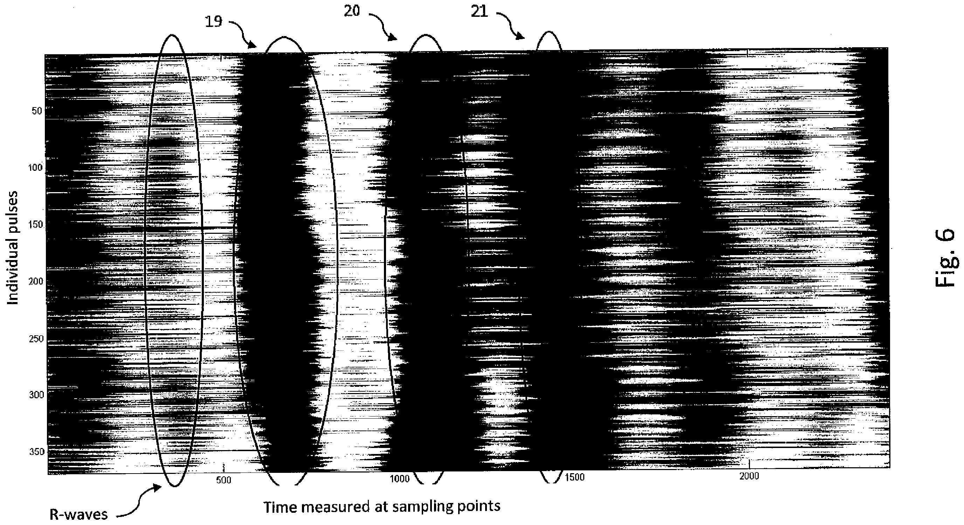

From Newton's laws of motion, specifically from the law of inertia, it follows that reflection of the pulse wave is accompanied by a mechanical movement of the head, which is measurable using the above described measuring mat 2. These mechanical head movements are depicted in Fig. 6, where they are identified in areas 19, 20 and 21.

The beginning of the mechanical pulse, which corresponds to the R-wave in the ECG signal, and $T_1$ of the pulse wave reflection 20 (mechanical head vibration) determines the time delay T between the R-wave and mechanical movement of the head 20. In the equation for the Windkessel model the R-wave represents the source part of the equation, which describes the activity of the piston pump corresponding to the heart 18. Blood is pumped into the brain under a pressure that equals ABP. The pressure of the brain tissue and cerebrospinal fluid that equals ICP acts against this pressure. The difference between ICP and the arterial pressure is called the cerebral perfusion pressure (CPP). The time delay T between the R-wave and reflection of the pulse wave in the head is then solved as a Winkessel equation using the relation II

$$CPP \sim -A \cdot log(T - T_0) \qquad \text{(II)}$$

where A is an empirically determined constant,
$T_0$ is the time that would correspond to the reflection time at infinite intracranial pressure. The time when the pulse wave appears on the carotid artery may be used as $T_0$.

In the following steps ICP is calculated using known ABP and CPP. This calculation is done using equation III

$$ABP - CPP = ICP \qquad\qquad (III)$$

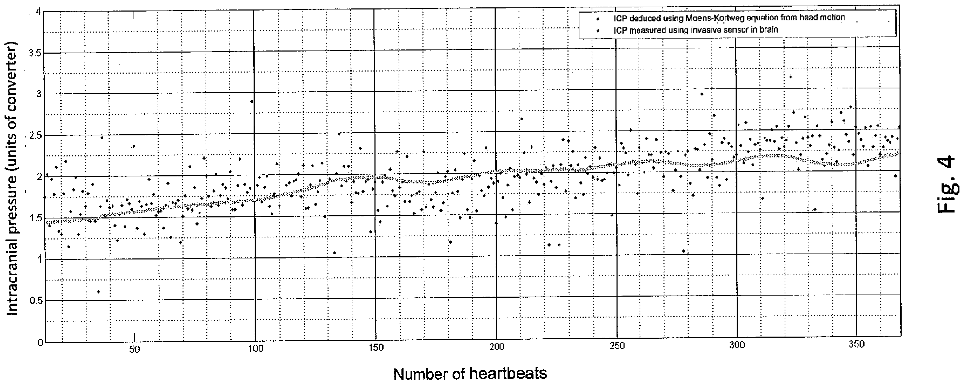

As is visible from Fig. 4, the trend of results measured using the described method is similar to the trend of results using an invasive method with intracranial sensors. The time delay $T$ between the head movement and ICP was calculated using equations II and III.

The non-invasive method was verified in several tested subjects. The verification equipment included an A/D converter 13, which was connected to the output of the invasive sensor for ICP measurement, output signal from ECG 4 and signal from the measuring mat 2 under the patient's head. All signals were sampled at frequency of 2 kHz.

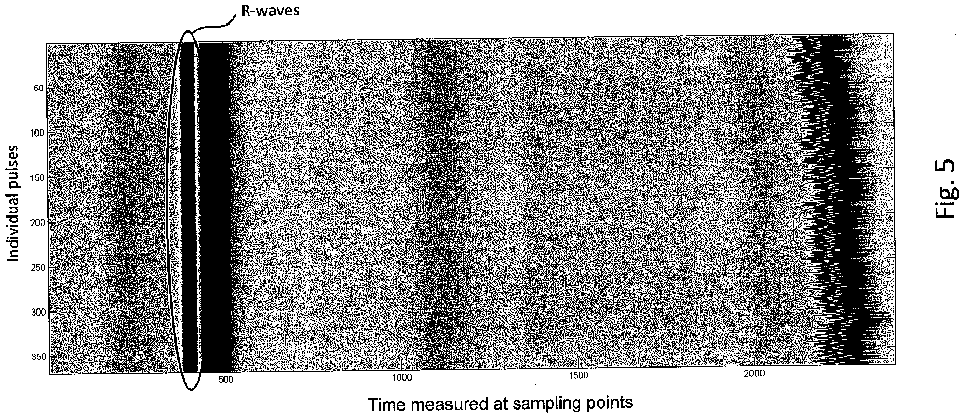

R-waves of the QRS complex were localized in the obtain ECG signal using standard procedures and the signal was then divided by R-waves into individual sections so that each section covered a time interval ($R_n$ - 400, $R_n$ +2000), where $R_n$ is the n-th R-wave and time is measured on sampling points, i.e., each such interval contains a section of signals that start 400 sampling points (0.2 s) before the R-wave and end 2000 sampling points (1 s) after the R-wave. The time range ($R_n$ - 400, $R_n$ + 2000) was selected for the verification experiment in order to ensure with absolute certainty that the given interval covers two consecutive R-waves. When these intervals are ordered under each other so that the first R-wave are synchronized in time, we get a data matrix for each measured channel, as shown in Fig. 5.

A signal from the piezoelectric sensor 8 corresponding to ordered R-waves is depicted in Fig. 6. Fig. 6 shows individual reflections of the pulse wave in the head represented by areas 19, 20, 21, caused by changes of the intracranial and arterial pressure. For purposes of this method, area 20 is the most important.

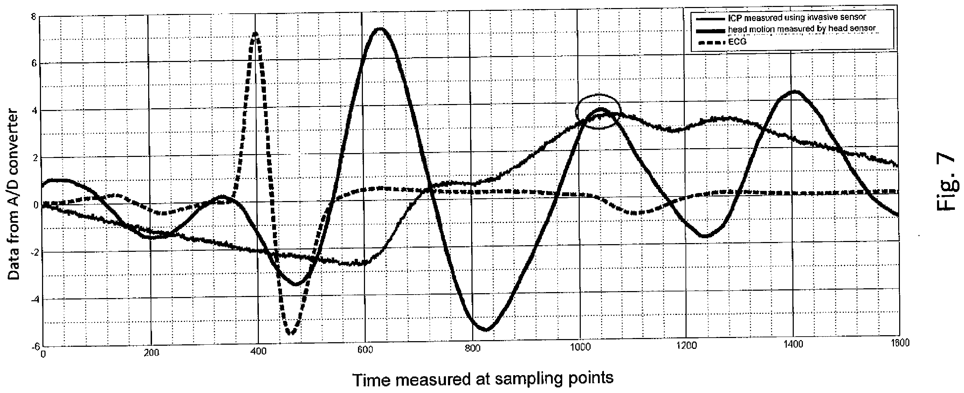

It may be assumed that during the time of ten consecutive R-waves ABP and ICP remain constant and the circulation system during these ten consecutive heart cycles remains stable. Fig. 7a and 7b show different values obtained from two different patients. As is clear from Fig. 7a and 7b, the time of maximum values of intracranial pressure matches the mechanical movement of the head 20 (movement). Data from the A/D converter were multiplied by suitable constants so that they can be displayed in a single graph.

METHOD II

The device for non-invasive monitoring of intracranial pressure 1 (ICP) includes a measuring mat 2, processor unit 3, displaying unit 6, network connector 7 and devices for measuring a parameter related to arterial blood pressure (ABP), which may be a device for recording electrical activity of the heart 4 or a device for invasive measurement of arterial blood pressure 5.

All calculations and relations are explained on a single hear cycle for the sake of simplicity. In terms of time, this concerns the area between two R-wave, where the first R-wave is considered the start of action $T$ (0). Practically immediately after the R-wave, a whole spectrum of mechanical actions occur in the bloodstream and they are reflected in the final signal in the form of oscillations, peaks, etc. For further processing it is necessary to identify only the peaks significant for further processing.

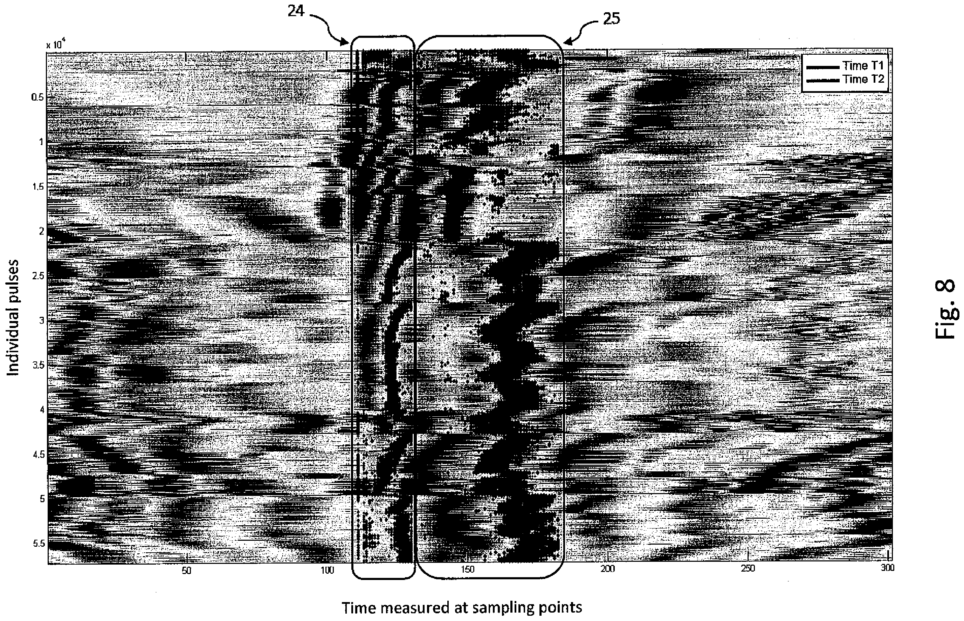

One of these key peaks is the one that corresponds to the moment the pulse wave is reflected from the head. This time is identified as $T_1$, the time is determined using the maximum value located in area 24, as is shown in Fig. 8. Blood is pumped into the brain at a pressure that equals ABP. The pressure of the brain tissue and cerebrospinal fluid that equals ICP acts against this pressure. There is a clear analogy with for example inflating a balloon using pressure $P_2$ inside a hollow sphere with a solid wall (similar to Fig. 3). If the balloon is inflated at a low pressure $P_2$, soon it couldn't increase its volume. If the pressure $P_2$ inflating the balloon is high, it would take longer to inflate and the amount of air in the balloon would be larger, proportional to pressure $P_2$. On the other hand, if there was higher pressure $P_1$ acting against pressure $P_2$ inflating the balloon, the balloon would stop inflating earlier as pressures $P_1$ and $P_2$ would balance earlier. It follows from this development and the analogical model that time $T_1$ inversely proportional to ICP. Equation IV therefore applies

$$T_1 \sim e^{-ICP} \qquad\qquad (IV)$$

The second key parameter is related to the closing of the aortic valve. This phenomenon is mechanically significant and it is known as the water hammer. This time is referred to as $T_2$, in Fig. 8 this moment corresponds to the peak in area 25. It was experimentally verified that this time correlates with the time when the pressure wave reaches sensor 5 for invasive measurement of ABP.

In accordance with the Moens-Korteweb equation in the following form (equation V)

$$ABP = \alpha \cdot ln\left(\frac{b}{\left(\frac{d}{PWV} - c\right)^2} - 1\right) \qquad (V)$$

where *ABP* refers to the arterial blood pressure;
*PWV* is the pulse wave velocity; and
*a, b, c* and d are certain constants,
it holds that $T_2$, when the pulse wave reached the place of measurement of ABP, is inversely proportionate to pressure, i.e, the higher the pressure, the lower the time of arrival of the pressure wave.

The specific equation for determining $T_2$ corresponds to the following equation (VI), as specified in the following text: F. Studnicka: Analysis of biomedical signals using differential geometry invariants, *ACTA PHYSICA POLONICA A,* 120, A-154, 2011.

$$ln(T_2) \sim - ABP \qquad (VI)$$

Some of the other results following from the Moens-Kortweg equation can be found for example in: E. Pinheiro, O. Postolache, P. Girão: Non-Intrusive Device for Real-Time Circulatory System Assessment with Advanced Signal Processing Capabilities, MEASUREMENT SCIENCE REVIEW, Volume 10, No. 5, 2010.

Time $T_0$ is introduced to the Windkessel model; this time is related to the moment the pulse wave reflects from the head. From the model it follows that ICP is proportionate to the logarithm of differences of times $T_1$ and $T_0$. Time $T_0$ can be substituted by time when the pulse wave goes through the carotid artery. This time correlates to the time when the pulse wave reaches the radial artery, i.e., the place where standard invasive measurements of ABP are taken using sensor 5. Again, $T_0$ in accordance with the Moens-Kortweg equation is inversely proportional to ABP.

Time $T_0$ could not be experimentally defined; however, it was possible to experimentally verify the correlation between $T_0$ and $T_2$. From the description above it follows that it is not necessary to measure ABP, only the time at which the pulse wave appear in the radial artery. This measurement can be carried out using a sensor for invasive measurement of ABP, as these measurements are carried out standardly in intensive care units. However, in order to detect relative changes of ICP, only data from measuring mat 2 are required, as documented by: F. Studnicka: Analysis of biomedical signals using differential geometry invariants, *ACTA PHYSICA POLONICA A,* 120, A-154, 2011.

From the equations stated above (IV and VI) and from the previous paragraph, we obtain equation VII

$$ICP \sim ln|(T_2 - T_1)| \qquad (VII)$$

This equation has been experimentally verified on tested subjects, even for situations when the head position changed.

On the basis of the description above, it is clear that for monitoring of relative changes of ICP it is possible to use the ICP monitoring device including only a measuring mat and a processor unit.

To measure absolute ICP values the device needs to include a measuring mat, processor unit and either a device for measurement of electric activities of the heart 4 or ABP monitoring device 5, or alternatively both.

The invention is defined in the appended clams.

List of reference numbers

**[0046]**

1    device for non-invasive ICP measurement
2    measuring mat
3    processor unit
4    device for measurement of electrical activity of the heart
5    device for invasive measurement of arterial pressure
6    displaying unit

| 7  | network connector |
|----|-------------------|
| 8  | sensor (in the mat) |
| 9  | extension |
| 10 | mattress |
| 11 | hospital bed |
| 12 | patient monitor |
| 13 | A/D converter |
| 14 | displaying device |
| 15 | system for collection of patient data |
| 16 | cranial cavity |
| 17 | bloodstream |
| 18 | heart |
| 19 | area I |
| 20 | area II |
| 21 | area III |
| 22 | maximum ICP |
| 23 | maximum mechanical manifestation |
| 24 | T1 area |
| 25 | T2 area |

**Claims**

1. A device (1) for non-invasive monitoring of intracranial pressure of a patient , wherein the device (1) comprises a measuring mat (2) comprising at least one sensor (8) for measuring mechanical manifestations of the head when the mat is placed under the patient's head; the device further comprising a reference device (4) whose output signal comprises an ECG signal; and a processor unit (3) configured for determining the time delay between a first signal corresponding to the R-wave in the ECG signal and a second signal corresponding to the mechanical manifestations of the head related to the reflection of the pulse wave in the bloodstream in the patient's head and for converting said time delay into the intracranial pressure of the patient.

2. The device (1) for non-invasive monitoring of intracranial pressure of the patient in accordance with claim 1 wherein the sensor (8) for measurement of mechanical manifestations of the head is a tensiometer.

3. The device (1) for non-invasive monitoring of intracranial pressure of the patient in accordance with claim 1 wherein the sensor (8) for measurement of mechanical manifestations of the head is an accelerometer.

4. The device (1) for non-invasive monitoring of intracranial pressure of the patient in accordance with claim 1 wherein the sensor (8) for measurement of mechanical manifestations of the head is a capacity sensor.

5. The device (1) for non-invasive monitoring of intracranial pressure of the patient in accordance with claim 1 wherein the processor unit (3) is connected to the measuring mat (2) for sending the first and second signal.

6. The device (1) for non-invasive monitoring of intracranial pressure of the patient in accordance with claim 1 wherein that the processor unit (3) is located inside the measuring mat (2).

7. The device (1) for non-invasive monitoring of intracranial pressure of the patient in accordance with claim 1 wherein the measured pressure inside the head is the intracranial pressure.

8. The device (1) for non-invasive monitoring of intracranial pressure of the patient in accordance with claim 1 wherein the calculated value of intracranial pressure is absolute

9. The device (1) for non-invasive monitoring of intracranial pressure of the patient in accordance with claim 1 wherein the processor unit (3) is connected to a displaying device (14).

10. The device (1) for non-invasive monitoring of intracranial pressure of the patient in accordance with claim 9 wherein the displaying device (14) is a part of the measuring device (1).

**11.** The device (1) for non-invasive monitoring of intracranial pressure of the patient in accordance with claim 9 wherein the displaying device (14) is a part of the patient monitor (12).

**12.** 44- The device (1) for non-invasive monitoring of intracranial pressure of the patient in accordance with claim 9 wherein the displaying device (14) is separate from the measuring device (1).

**13.** The device (1) for non-invasive monitoring of intracranial pressure of the patient in accordance with claim 9 wherein the displaying device (14) or the processor unit (3) is connected via a network connector (7) to the hospital system (15) for collection of patient data.

**14.** The device (1) for non-invasive monitoring of intracranial pressure of the patient in accordance with claim 9 wherein the displaying device (14) or the processor unit (3) is connected via wireless technology to the hospital system (15) for collection of patient data.

**15.** A method of using the device for non-invasive measurement of intracranial pressure of a patient of claim 1, wherein the processor unit (3) communicates with the measuring mat (2), the processor unit (3) records and stores the time of receiving the first signal and the time of receiving the second signal, and the processor unit (3) calculates the time delay between the first and the second signal and converts this delay to the intracranial pressure of the patient.

**16.** The method for non-invasive measurement of intracranial pressure of the patient in accordance with claim 15 wherein the measured pressure inside the head is the intracranial pressure.

**17.** Method for non-invasive measurement of intracranial pressure of the patient in accordance with claim 15 wherein the processor unit (3) communicates with the reference device (4) that sends information related to the blood pressure.

**18.** Method for non-invasive measurement of intracranial pressure of the patient in accordance with claim 17 wherein the processor unit (3) deduces the blood pressure value from the reference device (4).

**19.** The method for non-invasive measurement of intracranial pressure of the patient in accordance with claim 15 wherein the processor unit (3) calculates the absolute value of intracranial pressure depending on the value of the arterial blood pressure.

**Patentansprüche**

**1.** Gerät (1) für das nicht-invasive Monitoring des intrakraniellen Drucks (Hirndruck) des Patienten, **dadurch gekennzeichnet, dass** das Gerät die Messunterlage (2) mit zumindest einem Sensor (8) für das Messen mechanischer Erscheinungen des Kopfes umfasst, wobei sich die Messunterlage (2) unter dem Kopf des Patienten befindet; das Gerät umfasst ferner das Referenzgerät (4), dessen Ausgangssignal das EKG-Signal enthält, sowie die Prozessoreinheit (3), die für die Bestimmung der zeitlichen Verzögerung zwischen dem ersten, sich auf die R-Schwingung im EKG-Signal beziehenden Signal und dem zweiten, sich auf die mechanischen Erscheinungen des Kopfes im Zusammenhang mit der Reflexion der Pulswelle in der Blutbahn im Kopf des Patienten beziehenden Signal sowie für die Überführung dieser zeitlichen Verzögerung in den intrakraniellen Druck des Patienten konfiguriert ist.

**2.** Gerät (1) für das nicht-invasive Monitoring des intrakraniellen Drucks des Patienten gemäß dem Patentanspruch 1, **dadurch gekennzeichnet, dass** der Sensor (8) für das Messen der mechanischen Erscheinungen des Kopfes ein Dehnungsmessstreifen ist.

**3.** Gerät (1) für das nicht-invasive Monitoring des intrakraniellen Drucks des Patienten gemäß dem Patentanspruch 1, **dadurch gekennzeichnet, dass** der Sensor (8) für das Messen der mechanischen Erscheinungen des Kopfes ein Beschleunigungsmesser ist.

**4.** Gerät (1) für das nicht-invasive Monitoring des intrakraniellen Drucks des Patienten gemäß dem Patentanspruch 1, **dadurch gekennzeichnet, dass** der Sensor (8) für das Messen der mechanischen Erscheinungen des Kopfes ein Kapazitätssensor ist.

**5.** Gerät (1) für das nicht-invasive Monitoring des intrakraniellen Drucks des Patienten gemäß dem Patentanspruch 1, **dadurch gekennzeichnet, dass** die Prozessoreinheit (3) mit der Messunterlage (2) für das Senden des ersten und des zweiten Signals verbunden ist.

6. Gerät (1) für das nicht-invasive Monitoring des intrakraniellen Drucks des Patienten gemäß dem Patentanspruch 1, **dadurch gekennzeichnet, dass** sich die Prozessoreinheit (3) im Inneren der Messunterlage (2) befindet.

7. Gerät (1) für das nicht-invasive Monitoring des intrakraniellen Drucks des Patienten gemäß dem Patentanspruch 1, **dadurch gekennzeichnet, dass** der im Kopf gemessene Druck ein intrakranieller Druck ist.

8. Gerät (1) für das nicht-invasive Monitoring des intrakraniellen Drucks des Patienten gemäß dem Patentanspruch 1, **dadurch gekennzeichnet, dass** der errechnete Wert des intrakraniellen Drucks absolut ist.

9. Gerät (1) für das nicht-invasive Monitoring des intrakraniellen Drucks des Patienten gemäß dem Patentanspruch 1, **dadurch gekennzeichnet, dass** an die Prozessoreinheit (3) das Bildschirmgerät (14) angeschlossen ist.

10. Gerät (1) für das nicht-invasive Monitoring des intrakraniellen Drucks des Patienten gemäß dem Patentanspruch 9, **dadurch gekennzeichnet, dass** das Bildschirmgerät (14) Bestandteil des Messgerätes (1) ist.

11. Gerät (1) für das nicht-invasive Monitoring des intrakraniellen Drucks des Patienten gemäß dem Patentanspruch 9, **dadurch gekennzeichnet, dass** das Bildschirmgerät (14) Bestandteil des Patientenmonitors (12) ist.

12. Gerät (1) für das nicht-invasive Monitoring des intrakraniellen Drucks des Patienten gemäß dem Patentanspruch 9, **dadurch gekennzeichnet, dass** das Bildschirmgerät (14) vom Messgerät (1) getrennt ist.

13. Gerät (1) für das nicht-invasive Monitoring des intrakraniellen Drucks des Patienten gemäß dem Patentanspruch 9, **dadurch gekennzeichnet, dass** das Bildschirmgerät (14) oder die Prozessoreinheit (3) durch den Netzstecker (7) mit dem Krankenhaussystem (15) zur Erfassung der Patientendaten verbunden ist.

14. Gerät (1) für das nicht-invasive Monitoring des intrakraniellen Drucks des Patienten gemäß dem Patentanspruch 9, **dadurch gekennzeichnet, dass** das Bildschirmgerät (14) oder die Prozessoreinheit (3) mittels kabelloser Technologie mit dem Krankenhaussystem (15) zur Erfassung der Patientendaten verbunden ist.

15. Methode der Anwendung des Geräts für das nicht-invasive Monitoring des intrakraniellen Drucks des Patienten gemäß dem Patentanspruch 1, **dadurch gekennzeichnet, dass** die Prozessoreinheit (3) mit der Messunterlage (2) kommuniziert; die Prozessoreinheit (3) verzeichnet und speichert die Zeit des Empfangs des ersten Signals und die Zeit des Empfangs des zweiten Signals, wobei die Prozessoreinheit (3) die zeitliche Verzögerung zwischen dem ersten Signal und dem zweiten Signal berechnet und diese Zeitverzögerung in den intrakraniellen Druck des Patienten überführt.

16. Methode der Anwendung des Geräts für das nicht-invasive Monitoring des intrakraniellen Drucks des Patienten gemäß dem Patentanspruch 15, **dadurch gekennzeichnet, dass** der im Kopf gemessene Druck ein intrakranieller Druck ist.

17. Methode der Anwendung des Geräts für das nicht-invasive Monitoring des intrakraniellen Drucks des Patienten gemäß dem Patentanspruch15, **dadurch gekennzeichnet, dass** die Prozessoreinheit (3) mit dem Referenzgerät (4) kommuniziert, welches die Information bezüglich des Blutdrucks sendet.

18. Methode der Anwendung des Geräts für das nicht-invasive Monitoring des intrakraniellen Drucks des Patienten gemäß dem Patentanspruch 17, **dadurch gekennzeichnet, dass** die Prozessoreinheit (3) den Wert des Blutdrucks aus dem Referenzgerät (4) weiterleitet.

19. Methode der Anwendung des Geräts für das nicht-invasive Monitoring des intrakraniellen Drucks des Patienten gemäß dem Patentanspruch 15, **dadurch gekennzeichnet, dass** die Prozessoreinheit (3) den absoluten Wert des intrakraniellen Drucks in Abhängigkeit vom Wert des arteriellen Blutdrucks berechnet.

**Revendications**

1. L'équipement (1) pour le suivi non-invasif de la pression intracrânienne du patient **caractérisé en ce que** l'équipement comprend un tapis de mesure (2) avec au moins un capteur (8) pour mesurer les phénomènes mécaniques de la tête sachant que le tapis de mesure (2) est situé sous la tête du patient; l'équipement comprend ensuite un équipement de référence (4) dont le signal de sortie contient un signal ECG et une unité de calcul (3) configurée pour déterminer le retard entre le premier signal qui correspond à l'onde R du signal ECG et le second signal qui correspond à la manifestation mécanique émise par le retour de l'onde de pulsation dans le système sanguin de la tête du patient et pour convertir cet écart de temps en pression intracrânienne du patient.

2. L'équipement (1) pour le suivi non-invasif de la pression intracrânienne du patient selon la revendication 1 **caractérisé en ce que** le capteur (8) de mesure des manifestations mécaniques de la tête est extensométrique.

3. L'équipement (1) pour le suivi non-invasif de la pression intracrânienne du patient selon la revendication 1 **caractérisé en ce que** le capteur (8) de mesure des manifestations mécaniques de la tête est un accéléromètre.

4. L'équipement (1) pour le suivi non-invasif de la pression intracrânienne du patient selon la revendication 1 **caractérisé en ce que** le capteur (8) de mesure des manifestations mécaniques de la tête est un capteur capacitif.

5. L'équipement (1) pour le suivi non-invasif de la pression intracrânienne du patient selon la revendication 1 **caractérisé en ce que** l'unité de calcul (3) est connectée au tapis de mesure (2) pour l'envoi du premier et du deuxième signal.

6. L'équipement (1) pour le suivi non-invasif de la pression intracrânienne du patient selon la revendication 1 **caractérisé en ce que** l'unité de calcul (3) est située à l'intérieur du tapis de mesure (2).

7. L'équipement (1) pour le suivi non-invasif de la pression intracrânienne du patient selon la revendication 1 **caractérisé en ce que** la pression mesurée à l'intérieur de la tête est une pression intracrânienne.

8. L'équipement (1) pour le suivi non-invasif de la pression intracrânienne du patient selon la revendication 1 **caractérisée en ce que** la valeur calculée de la pression intracrânienne est absolue.

9. L'équipement (1) pour le suivi non-invasif de la pression intracrânienne du patient selon la revendication 1 **caractérisé en ce que** l'unité de calcul (3) est reliée au dispositif d'affichage (14).

10. L'équipement (1) pour le suivi non-invasif de la pression intracrânienne du patient selon la revendication 9 **caractérisé en ce que** le dispositif d'affichage (14) fait partie de l'équipement de mesure (1).

11. L'équipement (1) pour le suivi non-invasif de la pression intracrânienne du patient selon la revendication 9 **caractérisé en ce que** le dispositif d'affichage (14) fait partie du moniteur patient (12).

12. L'équipement (1) pour le suivi non-invasif de la pression intracrânienne du patient selon la revendication 9 **caractérisé en ce que** le dispositif d'affichage (14) est séparé de l'équipement de mesure (1).

13. L'équipement (1) pour le suivi non-invasif de la pression intracrânienne du patient selon la revendication 9 **caractérisé en ce que** le dispositif d'affichage (14) ou l'unité de calcul (3) est connecté par un connecteur de réseau (7) au système hospitalier (15) de collecte des données du patient.

14. L'équipement (1) pour le suivi non-invasif de la pression intracrânienne du patient selon la revendication 9 **caractérisé en ce que** le dispositif d'affichage (14) ou l'unité de calcul (3) est connecté par une technologie sans fil au système hospitalier (15) de collecte des données du patient.

15. La méthode d'utilisation de l'équipement de suivi non-invasif de la pression intracrânienne du patient selon la revendication 1 **caractérisée en ce que** l'unité de calcul (3) communique avec le tapis de mesure (2) ; l'unité de calcul (3) relève et enregistre le moment de la réception du premier signal et le moment de la réception du deuxième signal, et l'unité de calcul (3) calcule la différence de temps entre le premier signal et le deuxième signal, puis convertit ce retard en pression intracrânienne du patient.

16. La méthode d'utilisation de l'équipement de suivi non-invasif de la pression intracrânienne du patient selon la revendication 15 **caractérisée en ce que** la pression mesurée à l'intérieur de la tête est une pression intracrânienne.

17. La méthode d'utilisation de l'équipement de suivi non-invasif de la pression intracrânienne du patient selon la revendication 15 **caractérisée en ce que** l'unité de calcul (3) communique avec l'équipement de référence (4) qui envoie les informations concernant la pression sanguine.

18. La méthode d'utilisation de l'équipement pour le suivi non-invasif de la pression intracrânienne du patient selon la revendication 17 **caractérisée en ce que** l'unité de calcul (3) déduit la valeur de la pression sanguine à partir de l'équipement de référence (4).

19. La méthode d'utilisation de l'équipement de suivi non-invasif de la pression intracrânienne du patient selon la revendication 15 **caractérisée en ce que** l'unité de calcul (3) calcule la valeur absolue de la pression intracrânienne en fonction de la valeur de la pression artérielle.

Fig. 1a

Fig. 1b

Fig. 2a

Fig. 2b

Fig. 3

Fig. 4

Fig. 5

Fig. 6

Data from A/D converter

Fig. 7

18

Fig. 8

**EP 3 203 966 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013289422 A
- US 6761695 B
- US 2009012430 A
- US 2010049082 A
- US 8366627 B
- US 2013085400 A
- US 2011213254 A
- DE 3130326 A1
- EP 3054837 A
- US 10376216 B
- CN 105813551
- CZ 2014000112 W
- CZ PV2013781
- CZ 308222

**Non-patent literature cited in the description**

- **WAGSHUL M. et al.** The pulsating brain: A review of experimental and clinical studies of intracranial pulsatility. *Fluids and Barriers of the CNS,* 2011, vol. 8, 5
- **WESTERHOF N. et al.** *The arterial Windkessel,* 2008
- **E. PINHEIRO ; O. POSTOLACHE ; P. GIRÃO.** Non-Intrusive Device for Real-Time Circulatory System Assessment with Advanced Signal Processing Capabilities. *MEASUREMENT SCIENCE REVIEW,* 2010, vol. 10 (5